# EUROPEAN PATENT APPLICATION

(11) **EP 2 505 580 A1**
(43) Date of publication of application: **03.10.2012**
(21) Application number: 12161333.5
(22) Date of filing: 26.03.2012
(51) Int. Cl.: C07C 249/04, C07C 251/44

(54) **Method for producing oxime**

(30) Priority: 28.03.2011 JP 2011069446; 28.03.2011 JP 2011069448
(71) Applicant: Sumitomo Chemical Company, Limited, Tokyo 104-8260 (JP)
(72) Inventor: Tsujiuchi, Sho, Niihama-shi, Ehime 792-8521 (JP); Fukao, Masami, Niihama-shi, Ehime 792-8521 (JP)
(74) Representative: Duckworth, Timothy John

(57) **Abstract**

There is provided a method for producing an oxime compound, which is characterized by the steps of:
(a) oxidizing cumene to produce cumene hydroperoxide,
(b) subjecting the cumene hydroperoxide to an ammoximation reaction with ammonia and a ketone in the presence of a catalyst to produce a reaction mixture containing an oxime compound corresponding to the ketone and 2-phenyl-2-propanol,
(c) separating a fraction of 2-phenyl-2-propanol and the oxime compound corresponding to the ketone from the reaction mixture resulting in the step (b),
(d) converting 2-phneyl-2-propanol in the fraction to cumene, and
(e) recycling at least a portion of the cumene resulting in step(d) to step (a).

## Description

### BACKGROUND OF THE INVENTION

The present invention relates to a method for producing an oxime by ammoximation reaction of a ketone. The oxime is useful as a starting material of amide or lactam.

An ammoximation reaction of a ketone with cumene hydroperoxide and ammonia in the presence of a catalyst has been known (US 2010/324335A, and US 2010/01663A). In the disclosed methods, however, the production of the desired oxime is accompanied with 2-phenyl-2-propanol as a byproduct resulting from cumene hydroperoxide, and a satisfactory method with lesser waste of the byproduct was not known.

### SUMMARY OF THE INVENTION

The present invention provides a method for producing an oxime, which enables reuse of such byproduct by converting it to cumene and recycle the cumene to produce cumene hydroperoxide for use in the ammoximation reaction step.

The present invention relates to
1. a method for producing an oxime compound, which comprises the steps of:
   (a) oxidizing cumene to produce cumene hydroperoxide,
   (b) subjecting the cumene hydroperoxide to an ammoximation reaction with ammonia and a ketone in the presence of a catalyst to produce a reaction mixture containing an oxime compound corresponding to the ketone and 2-phenyl-2-propanol,
   (c) separating a fraction of 2-phenyl-2-propanol and the oxime compound corresponding to the ketone (hereinafter referred to also as "the oxime compound") from the reaction mixture resulting in step (b),
   (d) converting 2-phneyl-2-propanol in the fraction to cumene, and
   (e) recycling at least a portion of the cumene resulting in step (d) to step (a).

The present invention also includes following embodiments:
2. a method according to item 1 above, wherein the separated fraction of 2-phenyl-2-propanol in step (c) is a fraction that contains 4% or less by mass of the oxime compound corresponding to the ketone;
3. a method according to item 1 above, wherein the separated fraction of 2-phenyl-2-propanol in step (c) is a fraction that contains 1% or less by mass of the ammonia;
4. a method according to item 1 above, wherein the separated fraction of 2-phenyl-2-propanol in step (c) is a fraction that contains 1% or less by mass of the ammonia and 4% or less by mass of the oxime compound corresponding to the ketone;
5. a method according to item 1, 2,3 or 4 above, wherein the step (d) comprises following two steps of (d1) and (d2), or a step of (d3):
   (d1): supplying the fraction of 2-phenyl-2-propanol separated in step (c) to a dehydrating reaction in the presence of a dehydrating catalyst to produce α-methylstyrene, and
   (d2) supplying the resulting α-methylstyrene to hydrogenation reaction in the presence of a hydrogenation catalyst to produce cumene; or
   (d3) supplying the fraction of 2-phenyl-2-propanol separated in step (c) to hydrogenation reaction in the presence of a hydrogenation catalyst to produce cumene;
6. a method according to any one of items 1 to 5 above, wherein the catalyst is a catalyst that contains titan and silicon oxide;
7. a method according to any one of items 1 to 6 above, wherein the ketone is cycloalkanone; and
8. a method according to item 7 above, wherein the cycloalkanoe is cyclohexanone.

2-Phenyl-2-propanol resulting from ammoximation reaction can be recovered as a valuable material by converting the alcohol to cumene in an industrial production of an oxime by the present method.

### DESCRIPTION OF THE PREFERRED EMBODIMENTS

The oxidation of cumene is conducted by auto-oxidation comprising contacting cumene with an oxygen-containing gas. Examples of the sources of the oxygen-containing gas include air or pure oxygen gas, which may de diluted by inert gas, if necessary. The auto-oxidation may be conducted in the presence of an additive. Examples of the additive include, for example, bases. Examples of the base include, for example, alkali metal hydroxide such as sodium hydroxide, and potassium hydroxide; alkali metal carbonate such as sodium carbonate, and potassium carbonate; alkali metal hydrogen carbonate such as sodium hydrogen carbonate, and potassium hydrogen carbonate; ammonia, and ammonium carbonate. The oxidation of cumene may be conducted in the presence of a solvent.

The oxidation is preferably conducted at a temperature of 50 to 200°C. The oxidation is preferably conducted in a pressure range of from 0.1 to 5MPa(absolute).

The ketone that may be used in the present invention is typically an aliphatic ketone, an alicyclic ketone, unsaturated ketone, or an aromatic ketone. The ketones may be used alone or as a mixture of two or more of them. Examples of the aliphatic ketone include dialkyl ketones such as acetone, ethyl methyl ketone and isobutyl methyl ketone.

Examples of the unsaturated ketone include alkyl alkenyl ketones such as mesityl oxide.

Examples of the aromatic ketone include alkyl aryl ketones such as acetophenone; diaryl ketones such as benzophenone.

Examples of the alicyclic ketone include cycloalkanones such as cyclopentanone, cyclohexanone, cyclooctanone and cyclododecanone.

Examples of the unsaturated ketone also include cycloalkenones such as cyclopentenone and cyclohexenone. Among these ketones, cycloalkanones are preferably used in the present invention.

The ketone may be obtained by oxidation of an alkane, oxidation (dehydrogenation) of a secondary alcohol, or hydration and oxidation (dehydrogenation) of an alkene.

Ammonia may be used in the form of a gas, a liquid or a solution in an organic solvent. When gaseous ammonia is used, it may be diluted with an inert gas, if necessary.

Examples of the inert gas include, for example, nitrogen gas, carbon dioxide, helium, and argon. The amount of ammonia is preferably adjusted so that the concentration of ammonia in the liquid phase of the reaction mixture becomes 1% by mass or more. By adjusting the concentration of ammonia in the liquid phase of the reaction mixture to the predetermined value or more, the conversion rate of a ketone and the selectivity of an oxime can be increased, and thus the yield of an oxime as the objective product can also be increased. The concentration of ammonia is preferably 1.5% by mass or more, and usually 10% by mass or less, preferably 5% by mass or less. The amount of ammonia is preferably 1 mol or more, and more preferably 1.5 mol or more, per mol of the ketone.

The amount of cumene hydroperoxide, produced in step (a), that may be supplied to step (b) is preferably 0.5 to 20 moles, more preferably 0.5 to 15 moles per mol of the ketone.

Preferably, the catalyst that may be used in step (b) is a catalyst comprising titanium and silicon oxide, in view of oxime yield. Examples of the catalyst comprising titanium and silicon oxide include, for example, silicate containing titanium, and silica containing titanium.

Examples of the silicate containing titanium include, for example, mesoporous silicate containing titanium, and crystalline silicate containing titanium. Examples of the mesoporous silicate include, for example, MCM-41, MCM-48, HMS, SBA-15, FSM-16, MSU-H, and MSU-F. Examples of the crystalline silicate containing titanium include, for example, silicalite-1(MFI-type), silicalite-2(MEL-type), ITQ-1(MWW-type), and YNU-2(MSE-type). Among the silicate containing titanium, preferred is mesoporous silicate containing titanium, and more preferred are MCM-41 containing titanium, which may be also hereinafter referred to as Ti-MCM-41, and HMS containing titanium, which may be also hereinafter referred to as Ti-HMS.

The "mesoporous silicate" herein means a silicate having mesopores of which pore-diameter is approximately 2 to approximately 50 nm. The presence of the mesoporous structure can be confirmed by the presence of a peak(s) at 2θ = 0.2 to 4.0° in XRD(X-ray diffraction) analysis using copper-Kα beam. The titanium contained in the silicate containing titanium may be incorporated into the silicate framework, or pores, or may be supported on the surface of the silicate framework. Preferred are the silicates containing titanium in the framework of the silicate such as the mesoprous silicate, crystalline silicate, MCM-41, and HMS, all of which contain titanium in the framework of the silicate.

Examples of the silica containing titanium include titanium-supported silica carrier, and a composite oxide of titania-silica.

The catalyst containing titanium and silicon oxide may contain further element(s) such as boron, aluminum, gallium, iron, or chromium. The silicate containing titanium may contain such element(s) in the framework of the silicate, in the pores, or on the surface of the framework. Examples of the titanosilicate containing titanium in the silicate framework include a titanosilicate containing titanium, silicon and oxygen as the elements that constitute the framework, and a titanosilicate having a framework consisting essentially of titanium, silicon, and oxygen, or a titanosilicate containing boron, aluminum, gallium, iron, and chromium, other than titanium, silicon and oxygen atoms. Silica containing titanium, as the catalyst, may contain other elements incorporated in the silica framework or supported on the silica surface.

Titanium content in the catalyst containing titanium and silicon oxide is preferably 0.0001 or more, preferably 0.005 or more in terms of Ti/Si, and is preferably 1.5 or less, more preferably 1.0 or less. The amount of the elements other than titanium, silicon and oxygen in the catalyst is expressed by the ratio between the element and the silicon atoms (element/Si), and such ratio is preferably 1.0 or less, more preferably 0.5 or less. Oxygen may be present in the catalyst in such amount that balances the contents and oxidation number of other elements. A typical composition of the catalyst comprising titanium and silicon oxide is expressed by the following formula normalized to the element silicon:

SiO₂·xTiO₂·yMnO_{n/2}

wherein M represents an element(s) other than silicon, titanium, and oxygen, n represents an oxidation number of the element M, x is 0.0001 to 1.0, and y is 0 to 1.0.

Examples of the element M include, for example, boron, aluminum, gallium, iron, and chromium.

The catalyst containing titanium and silicon oxide can be prepared by a known hydrothermal reaction or a sol-gel method. Such catalyst may be used after being treated by contacting with a silicon compound. Examples of the silicon compound include, for example, an organic silicon compound, and an inorganic silicon compound. Preferred is the organic silicon compound.

The catalyst that may be used in step (b) is preferably used in a form of particles or pellets by molding optionally with a binder, or may be used after supported on a carrier.

A solvent may be used in the ammoximation reaction. Examples of the solvent include, hydrocarbon such as butane, pentane, hexane, cyclohexane, benzene, cumene, toluene, and xylene, nitriles such as acetonitrile, propionitrile, butyronitrile, isobutyronitrile, trimethylacetonitrile, valeronitrile, isovaleronitrile and benzonitrile; and alcohols such as methyl alcohol, ethyl alcohol, n-propyl alcohol, isopropyl alcohol, n-butyl alcohol, s-butyl alcohol, t-butyl alcohol and t-amyl alcohol, and still more preferably a nitrile or alcohol having up to 2 carbon atoms. If necessary, two or more kinds of them can be used. In the present invention, the water content in the liquid phase of the reaction mixture is preferably kept lower in view of the selectivity of the oxime.

The amount of the solvent is usually from 1 to 500 parts by weight, and preferably from 2 to 300 parts by weight, per 1 part by weight of the ketone.

The amount of the cumene hydroperoxide that may be used is usually from 0.5 to 20 moles, and preferably from 0.5 to 15 moles, per 1 mol of a ketone.

Next, the mode of the ammoximation reaction will be explained. In the present invention, first, a solvent, a titanosilicate and an organic peroxide are introduced to a reactor. There is no particular limitation with respect to the order of introduction of them. Then, ketone and ammonia are typically fed to the reactor in which the titanosilicate is suspended by stirring. The ketone and ammonia are typically fed simultaneously as a ketone feed and an ammonia feed, which is referred to as "co-feed" in this specification, or as a mixture thereof. Preferably, a portion of ammonia may be preliminarily charged or fed together with the organic peroxide, and then the ketone and the remaining ammonia may be fed to the reactor. Alternatively, the organic peroxide may be preliminarily fed to the reactor and then the ketone, ammonia, and additional organic peroxide may be fed together.

The ammoximation reaction may be carried out batch-wise or continuously. Particularly preferred is a continuous reaction process which comprises withdrawing a liquid phase of the resulting reaction mixture containing the product, and feeding reactants, typically ketone and ammonia, simultaneously in view of productivity and operability.

For example, a continuous reaction is preferably carried out by preparing a reaction mixture in which titanosilicate is suspended in a reactor, feeding reaction starting materials such as ketone and ammonia to the reactor, and withdrawing a liquid phase of the reaction mixture from the reactor through a filter.

Preferably employed is a reactor lined with glass or made of stainless steel for preventing of decomposition of the organic peroxide.

The temperature of the ammoximation reaction is preferably from 50 to 200°C, and more preferably from 80 to 150°C. The reaction pressure may be normal pressure, and is usually from 0.2 to 1 MPa (absolute pressure), and preferably from 0.2 to 0.5 MPa (absolute pressure), so as to readily dissolve ammonia in the liquid phase of the reaction mixture. The reaction pressure may be adjusted by using an inert gas such as nitrogen or helium.

The post-treatment operation of the resulting reaction mixture is appropriately selected. For example, an oxime can be separated by separating a titanosilicate from the reaction mixture through filtration or decantation and distillating a obtained liquid phase.

The separating of a fraction of 2-phenyl-2-propanol in step (c) from a reaction mixture containing the oxime compound and 2-phenyl-2-propanol resulting from the ammoximation reaction is conducted, for example, by distillation of a liquid phase obtained by removing the catalyst from the reaction mixture resulting from step (b). Alternatively, 2-phenyl-2-propnaol is extracted from the liquid phase, or the oxime compound is removed by crystallization from the liquid phase. The distillation, extraction and/or crystallization procedures may be optionally combined for the separation. Preferably employed is a fraction of 2-phenyl-2-propanol that was separated in such a way that the fraction contains the oxime compound in an amount of 4% or less by mass of the fraction. More preferred is a fraction of 2-phenyl-2-propanol that contains the oxime compound in an amount of 3.5% or less by mass of the fraction, which can be obtained in a similar manner. The separated catalyst can be reused by washing, calcination, and a contact treatment with a silicon compound.

The separated fraction of 2-phenyl-2-propanol as above may contain unreacted ammonia used in the ammoximation reaction, and the concentration of ammonia in the separated fraction of 2-phenyl-2-propanol is preferably adjusted to 1% or less by mass. Typical procedures therefore include, for example, distillation, or stripping of ammonia by blowing an inert gas such as nitrogen gas, whereby ammonia is removed, from the liquid phase, or washing with acid such as phosphoric acid, or sulfuric acid, or contact-treatment with a solid acid substance such as ion-exchange membranes, ion-exchange resins, or activated clay, whereby adsorbing ammonia. Thus, the separated fraction of 2-phenyl-2-propanol in step (c) preferably contains the oxime compound in an amount of 4% or less by mass, more preferably in an amount of 3.5% or less together with ammonia in an amount of 1% or less by mass of the fraction. Such fractions are preferably used in step (d-1), or step (d-3) of the invention.

The converting of 2-phneyl-2-propanol in the fraction to cumene is conducted, for example, by (d-1): supplying the fraction of 2-phenyl-2-propanol separated in step (c) to a dehydrating reaction in the presence of a dehydration catalyst to produce α-methylstyrene, and
(d-2) supplying the resulting α-methylstyrene to hydrogenation reaction in the presence of a hydrogenation catalyst to produce cumene; or
(d-3) supplying the fraction of 2-phenyl-2-propanol separated in step (c) to hydrogenation reaction in the presence of a hydrogenation catalyst to produce cumene.

Examples of the dehydration catalyst employed in step of (d1) include, for example, inorganic acid such as sulfuric acid, or phosphoric acid, an organic acid such as methanesulfonic acid, and p-toluenesulfonic acid, a solid acid such as activated alumina, titania, zirconia, and zeolite. Preferred is the solid acid, more preferred is activated alumina in view of selectivity to α-methylstyrene, catalyst life, and readily separable nature of the catalyst after reaction.

The dehydration is preferably conducted at a temperature of from 50 to 450°C, and in a pressure range of from 0.01 to 10 MPa (absolute pressure). The dehydration reaction may be conducted in the presence of hydrogen, which will be used in the following hydrogenation step. α-Methylstyrene formed in this step may contain water formed by dehydration, which can be supplied to the following step as it is or may be supplied to the following step after the water is separated.

Examples of the hydrogenation catalyst include, for example, a catalyst comprising a metal such as nickel, palladium, platinum, copper or metal compounds thereof. Among them, a catalyst comprising palladium or a palladium compound, and a catalyst comprising copper or a copper compound, are preferred, in view of selectivity to cumene and suppressed hydrogenation of α-methylstyrene nucleus.

Examples of the catalyst comprising palladium or palladium compound include, for example, a palladium-supported catalyst. Examples of the catalyst comprising copper or copper compound include, for example, a copper-supported catalyst.

Examples of carriers that may be used for the supported catalyst include, for example, alumina, silica, titania, magnesia and active carbon. Two or more of the hydrogenation catalysts may be used together.

The hydrogenation step is conducted preferably at a temperature range of from 0 to 500°C, more preferably from 30 to 300°C. The hydrogenation is preferably conducted in a range of from 0.1 to 10MPa (absolute). Hydrogen is used preferably in the amount of 1 to 10 moles, more preferably 1 to 5 moles per mol of α-methylstyrene.

The reaction of steps (d-1) and (d-2) can be carried out as a single step in the presence of a suitable catalyst capable of catalyzing both of the dehydration and hydrogenation reactions.

The reaction of steps (d-1) and (d-2) may be carried out in a batch-wise or continuous or semi-batch-wise manner, or the reactions can be conducted in a manner by combining these. Particularly preferred is a continuous reaction process, which is more preferably conducted by fixed-bed type flow reactor by flowing reaction materials through the fixed-bed type reactor where catalysts are charged, in view of productivity and operability. The fixed-bed type flow reaction can be conducted, for example, by supplying reaction materials as up-flow stream, dawn-flow stream, or countercurrent-flow stream. The reaction of steps (d-1) and (d-2) may be carried out by using independent fixed-bed type reactor or a single fixed-bed type reactor.

### EXAMPLES

The present invention will be explained by way of the following examples and comparative examples, but it is not to be construed to limit the present invention thereto. In the following examples, the liquid phase of the reaction mixture was analyzed by gas chromatography, and the conversion rate of cyclohexane as well as the selectivity and yield of cyclohexanone oxime were calculated based on the results of the analysis.

### Example 1

In a 1 L autoclave (reactor), 100 g of a toluene solution containing 0.3% by mass of cyclohexanone oxime, and 10% by mass of 2-phenyl-2-propanol, and 3.0g of palladium/alumina catalyst were charged. The atmosphere of the reactor was replaced with nitrogen gas, and then pressurized with hydrogen gas by 0.9 MPa (absolute pressure), warmed to 200°C under stirring, and thereafter reacted for 6 hrs. The reaction solution was withdrawn and analyzed by gas-chromatography, which revealed that conversion rate of 2-phenyl-2-propanol was 99.6%, selectivity to cumene was 88.5% and cumene yield was 88.1%.

### Example 2

In a 1 L autoclave (reactor), 100 g of a toluene solution containing 0.05% by mass of ammonia, and 10% by mass of 2-phenyl-2-propanol, and 3.0g of palladium/alumina catalyst were charged. The atmosphere of the reactor was replaced with nitrogen gas, and then pressurized with hydrogen gas by 0.9 MPa (absolute pressure), warmed to 200°C under stirring, and thereafter reacted for 6 hrs. The reaction solution was withdrawn and analyzed by gas-chromatography, which revealed that conversion rate of 2-phenyl-2-propanol was 99.9%, selectivity to cumene was 91.3% and cumene yield was 91.2%.

### Comparative Example 1

In a 1 L autoclave (reactor), 100 g of a toluene solution containing 0.5% by mass of cyclohexanone oxime, and 10% by mass of 2-phenyl-2-propanol, and 3.0g of palladium/alumina catalyst were charged. The atmosphere of the reactor was replaced with nitrogen gas, and then pressurized with hydrogen gas by 0.9 MPa (absolute pressure), warmed to 200°C under stirring, and thereafter reacted for 6 hrs. The reaction solution was withdrawn and analyzed by gas-chromatography, which revealed that conversion rate of 2-phenyl-2-propanol was 19.3%, selectivity to cumene was 79.0% and cumene yield was 15.2%.

### Comparative Example 2

In a 1 L autoclave (reactor), 100 g of a toluene solution containing 0.13% by mass of ammonia, and 10% by mass of 2-phenyl-2-propanol, and 3.0g of palladium/alumina catalyst were charged. The atmosphere of the reactor was replaced with nitrogen gas, and then pressurized with hydrogen gas by 0.9 MPa (absolute pressure), warmed to 200°C under stirring, and thereafter reacted for 6 hrs. The reaction solution was withdrawn and analyzed by gas-chromatog-raphy, which revealed that conversion rate of 2-phenyl-2-propanol was 29.4%, selectivity to cumene was 96.3% and cumene yield was 28.2%.

## Claims

1. A method for producing an oxime compound, which comprises the steps of:
(a) oxidizing cumene to produce cumene hydroperoxide,
(b) subjecting the cumene hydroperoxide to an ammoximation reaction with ammonia and a ketone in the presence of a catalyst to produce a reaction mixture comprising an oxime compound corresponding to the ketone and 2-phenyl-2-propanol,
(c) separating a fraction of2-phenyl-2-propanol and the oxime compound corresponding to the ketone from the reaction mixture resulting in step (b),
(d) converting 2-phenyl-2-propanol in the fraction to cumene; and
(e) recycling at least a portion of the cumene resulting in step (d) to step (a).

2. A method according to claim 1, wherein the separated fraction of 2-phenyl-2-propanol in step (c) is a fraction that contains 4% or less by mass of the oxime compound corresponding to the ketone.

3. A method according to claim 1, wherein the separated fraction of 2-phenyl-2-propanol in step (c) is a fraction that contains 1% or less by mass of ammonia.

4. A method according to claim 1, wherein the separated fraction of 2-phenyl-2-propanol in step (c) is a fraction that contains 1% or less by mass of the ammonia and 4% or less by mass of the oxime compound corresponding to ketone.

5. A method according to claim 1, 2, 3 or 4, wherein the step (d) comprises two steps (d-1) and (d-2):
(d-1): supplying the fraction of 2-phenyl-2-propanol separated in step (c) to a dehydrating reaction in the presence of a dehydrating catalyst to produce α-methylstyrene, and
(d-2): supplying the resulting α-methylstyrene to a hydrogenation reaction in the presence of a hydrogenation catalyst to produce cumene.

6. A method according to claim 1, 2, 3 or 4 wherein the step (d) comprises a step (d-3):
(d-3): supplying the fraction of 2-phenyl-2-propanol separated in step (c) to a hydrogenation reaction in the presence of a hydrogenation catalyst to produce cumene.

7. A method according to any one of claims 1 to 6, wherein the catalyst in step (b) is a catalyst that comprises titanium and silicon oxide.

8. A method according to any one of claims 1 to 7 wherein the ketone is a dialkyl ketone, an alkyl alkenyl ketone, an alkyl aryl ketone, a diaryl ketone, a cycloalkanone, a cycloalkenone or a mixture thereof, wherein said alkyl groups contain from 1 to 10 carbon atoms, said alkenyl group contains from 2 to 10 carbon atoms, said alicyclic groups contain from 5 to 10 carbon atoms in the ring and said aryl groups contain from 6 to 10 carbon atoms in the aromatic ring system.

9. A method according to any one of claims 1 to 7, wherein the ketone is a cycloalkanone.

10. A method according to claim 9, wherein the cycloalkanone is cyclohexanone.
